(19) Europäisches Patentamt
European Patent Office
Office européen des brevets

(11) **EP 4 079 346 B1**

(12) **EUROPEAN PATENT SPECIFICATION**

(45) Date of publication and mention
of the grant of the patent:
**10.09.2025   Bulletin 2025/37**

(21) Application number: **20904051.8**

(22) Date of filing: **30.11.2020**

(51) International Patent Classification (IPC):
*A61M 5/142* (2006.01)      *A61M 5/168* (2006.01)

(52) Cooperative Patent Classification (CPC):
**A61M 5/14244; A61M 5/1452;** A61M 5/1422;
A61M 2005/14252; A61M 2205/106;
A61M 2205/3327; Y02E 60/10

(86) International application number:
**PCT/KR2020/017238**

(87) International publication number:
**WO 2021/125617 (24.06.2021 Gazette 2021/25)**

(54) **LIQUID MEDICINE INJECTION DEVICE HAVING DRIVING TIME SYMMETRIZATION ALGORITHM APPLIED THERETO, DRIVING TIME SYMMETRIZATION METHOD, AND RECORDING MEDIUM THEREOF**

VORRICHTUNG ZUR INJEKTION EINER FLÜSSIGEN ARZNEI MIT EINEM DARAUF ANGEWANDTEN ANSTEUERZEITSYMMETRISIERUNGSALGORITHMUS, VERFAHREN ZUR SYMMETRISIERUNG DER ANSTEUERZEIT UND AUFZEICHNUNGSMEDIUM DAFÜR

DISPOSITIF D'INJECTION DE MÉDICAMENT LIQUIDE AYANT UN ALGORITHME DE SYMÉTRISATION DE TEMPS D'ENTRAÎNEMENT APPLIQUÉ À CELUI-CI, PROCÉDÉ DE SYMÉTRISATION DE TEMPS D'ENTRAÎNEMENT ET SUPPORT D'ENREGISTREMENT DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **17.12.2019   KR 20190169187**

(43) Date of publication of application:
**26.10.2022   Bulletin 2022/43**

(73) Proprietor: **Eoflow Co., Ltd.
Bundang-gu, Seongnam-si
Gyeonggi-do 13605 (KR)**

(72) Inventors:
• **KIM, Seon Hwan
Seongnam-si Gyeonggi-do 13604 (KR)**

• **PARK, Eun Sung
Seoul 08063 (KR)**
• **HAN, Yong Joon
Seoul 05266 (KR)**

(74) Representative: **Mewburn Ellis LLP
Aurora Building
Counterslip
Bristol BS1 6BX (GB)**

(56) References cited:
WO-A1-2008/107467      JP-A- 2013 212 415
JP-A- H0 531 175      KR-A- 20010 042 349
KR-A- 20110 126 014      KR-A- 950 014 579
US-A- 5 807 075      US-A1- 2007 104 596

**Description**

TECHNICAL FIELD

The present disclosure relates to a drug injection device including a pump module and a recording medium of a driving time symmetrization method. More particularly, the present disclosure relates to a drug injection device to which a driving time symmetrization algorithm is applied and a recording medium of the driving time symmetrization method.

BACKGROUND ART

Diabetes mellitus is a disease based on metabolic abnormalities caused by insufficient insulin, one of the hormones secreted by the pancreas. Diabetic patients can use a method of injecting insulin into the body as one of the active methods. An insulin injection device (hereinafter referred to as a 'drug injection device') may be used to appropriately inject insulin into the body in accordance with changes in blood sugar of a patient.

In regard to an electroosmotic pressure-based actuator (hereinafter referred to as a 'pump module') included in the drug injection device, the driving time of a pull operation and a push operation thereof to reach forward and backward points varies according to the characteristics of the pump module. The characteristics of the pump module may include a friction force, length, temperature, load, electrolysis, and the like.

When the driving times of the pull operation and the push operation are asymmetric, gas is generated inside the pump module, degrading the performance and shortening the product lifespan thereof as time passes.

US 2007/104596 A1 proposes a pump assembly comprising an actuator lever, a supporting structure, a pump comprising a pump member moveable by actuation of the actuator lever, an actuator for moving the actuator lever, a first stationary pivoting joint formed between the actuator lever and the supporting structure, and a second floating pivoting joint is formed between the actuator lever and the pump member allowing the pump member to float relative to the actuator lever, the floating pivoting point providing a constant-length actuator arm defined between the first pivoting joint and the second pivoting joint.

WO 2008/107467 A1 proposes a development of the pump assembly in US 2007/104596 A1, wherein the actuator lever and the actuator are coupled to each other by a coupling joint arranged between the first and the second pivoting joint in such a way that rotation of the actuator in a first direction causes the actuator lever to rotate in an opposite second direction, thereby providing as system which can be made less susceptible to the influence of acceleration.

DESCRIPTION OF EMBODIMENTS

TECHNICAL PROBLEM

One or more embodiments include a drug injection device including a pump module having a symmetritized driving time, and a recording medium of a driving time symmetrization method.

However, the objectives are exemplary, and the scope of the present disclosure is not limited thereto.

SOLUTION TO PROBLEM

According to a first aspect, the present invention provides a drug injection device according to claim According to a second aspect, the present invention provides a non-transitory computer-readable recording medium according to claim 3.

Other aspects, features and advantages other than those described above will become apparent from the following detailed description, claims and drawings for carrying out the invention.

ADVANTAGEOUS EFFECTS OF DISCLOSURE

As described above, according to a driving time symmetrization algorithm of the present disclosure, gas inside an electroosmotic pressure-based actuator is not generated due to the symmetrical driving time, thus increasing the lifespan thereof.

The scope of the present disclosure, however, is not limited by the effects described above.

BRIEF DESCRIPTION OF DRAWINGS

FIG. 1 is a perspective view of a drug injection device according to an embodiment of the present disclosure.

FIG. 2 is a perspective view of an internal arrangement of the drug injection device of FIG. 1.

FIG. 3 is a plan view of the drug injection device of FIG. 2.

FIG. 4 is a perspective view of a pump module according to an embodiment of the present disclosure, and FIG. 5 is a cross-sectional view the at least one sensor taken along line II-II' of FIG. 4.

FIGS. 6A and 6B are schematic diagrams illustrating reactions in first and second electrode bodies with respect to a membrane, according to an embodiment of the present disclosure.

FIGS. 7A and 7B are cross-sectional views illustrating a reciprocating motion of a shaft according to an embodiment of the present disclosure.

FIG. 8A is a plan view for describing a drug injection device according to an embodiment of the present disclosure, the drug injection device injecting a drug through a pump module and a driving unit.

FIG. 8B is a perspective view for describing a drug injection device according to an embodiment of the present disclosure, the drug injection device injecting a drug through a pump module and a driving unit.

FIG. 9 is a plan view for describing an operation of a driving unit according to an embodiment of the present disclosure.

FIG. 10 is a simple flowchart for describing a driving time symmetrization method of a drug injection device, the method to be implemented according to an embodiment of the present disclosure.

FIG. 11 is a flowchart of a method performed by a drug injection device, the method to be implemented according to an embodiment of the present disclosure, of applying an additional driving time.

FIG. 12 is a flowchart of another method, performed by a drug injection device, the method to be implemented according to an embodiment of the present disclosure, of applying an additional driving time.

FIGS. 13A to 13C illustrate the effect of symmetrical driving time of a pump module, to which a symmetrization algorithm according to an embodiment of the present disclosure is applied.

## MODE OF DISCLOSURE

Hereinafter, various embodiments of the present disclosure are described in connection with the accompanying drawings. As the present disclosure allows for various changes and many different forms, particular embodiments will be illustrated in the drawings and described in detail in the written description. However, this is not intended to limit the present disclosure to particular modes of practice, and it is to be appreciated that changes that do not depart from the technical scope of the present disclosure are encompassed in the present disclosure. In regard to the description of the drawings, like reference numerals denote like elements.

In various embodiments of the present disclosure, it is to be understood that the terms such as "including" or "having," etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

In various embodiments of the present disclosure, the terms such as "or" include any and all combinations of words listed together. For example, "A or B" may include A, B, or both A and B.

The terms such as "first", "second", etc. used in various embodiments of the present disclosure may modify various components of the various embodiments, but they do not limit the components. For example, the terms above do not limit the order and/or importance of the components, and may be used to distinguish one component from another.

When an element is "connected" or "coupled" to another element, it may be construed that the element is connected or coupled to the other element not only directly but also through at least one of other elements interposed therebetween

In the embodiments of the present disclosure, the terms such as "module", "unit", "part", etc. are terms for referring to an element performing at least one function or operation, and such element may be implemented by hardware or software or a combination of hardware and software. In addition, a plurality of "modules", "units", "parts", etc. may be integrated into at least one module or chip and thus as at least one processor, except when each of them needs to be implemented by certain individual hardware.

Terms as defined in a commonly used dictionary should be construed as having the same meaning as in an associated technical context, and unless defined apparently in various embodiments of the present disclosure, the terms are not ideally or excessively construed as having formal meaning.

Hereinafter, various embodiments of the present disclosure will be described in detail with reference to the accompanying drawings.

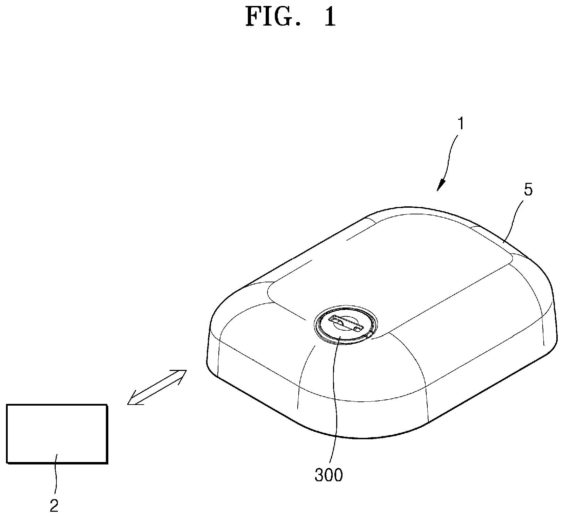

FIG. 1 is a perspective view of a drug injection device 1 according to an embodiment of the present disclosure.

Referring to FIG. 1, the drug injection device 1 may be attached to a drug injection object, and may be used to inject a prescribed dosage of a drug stored therein, for a user. In a selective embodiment, the drug injection device 1 may be mounted on the bod of the user. In addition, in another selective embodiment, the drug injection device 1 may be mounted on an animal to inject a drug to the animal.

The drug injection device 1 may be used for various purposes according to the type of a drug to be injected. For example, the drug may include an insulin-based drug for diabetic patients, and may include other drugs for the pancreas, a drug for the heart, and other various types of drugs.

The drug injection device 1 may be connected to a remote device 2 connected thereto by wire or wirelessly. The user may operate the remote device 2 to use the drug injection device 1, and may monitor a usage state of the drug injection device 1. For example, the amount of drug injected from the drug injection device 1, the number of injections of the drug, the amount of drug stored in a reservoir 200, bio-information of the user, etc. may be monitored, and the user may drive the drug injection device 1 based on information about the monitoring.

In an embodiment, the remote device 2 refers to a communication terminal capable of using an application in a wired/wireless communication environment. Here, the remote device 2 may be a portable terminal of the user. In more detail, the remote device 2 may include a computer (e.g., desktop, laptop, tablet, etc.), a media computing platform (e.g., cable, satellite set-top box, digital video recorder), a handheld computing device (e.g., a personal digital assistant (PDA), an e-mail client, etc.), any type of mobile phone, any type of mobile phone, any type of wearable device that may be attached to or mounted on the body of the user, or any type of other type of computing or communication platform, but the present disclosure is not limited thereto.

The drug injection device 1 and the remote device 2 may communicate with each other via a communication network. In this case, the communication network refers to a communication network that provides a connection path for the remote device 2 to transmit and receive data after accessing a service server (not shown). Examples of the communication network may include wired networks such as Local Area Networks (LANs), Wide Area Networks (WANs), Metropolitan Area Networks (MANs), Integrated Service Digital Networks (ISDNs), and wireless networks such as wireless LANs, code-division multiple access (CDMA), Bluetooth, and satellite communication, or the like, but the present disclosure is not limited thereto.

Referring to FIG. 1, the remote device 2 is illustrated as a single device, but the present disclosure is not limited thereto, and the remote device 2 may include a plurality of devices capable of communicating with the drug injection device 1.

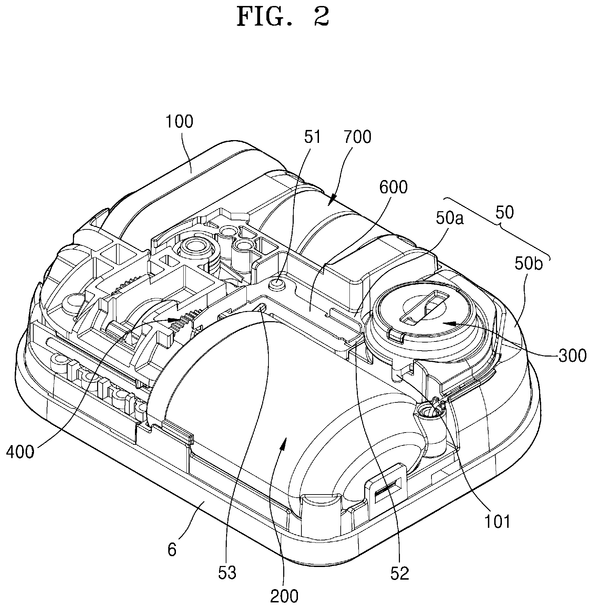

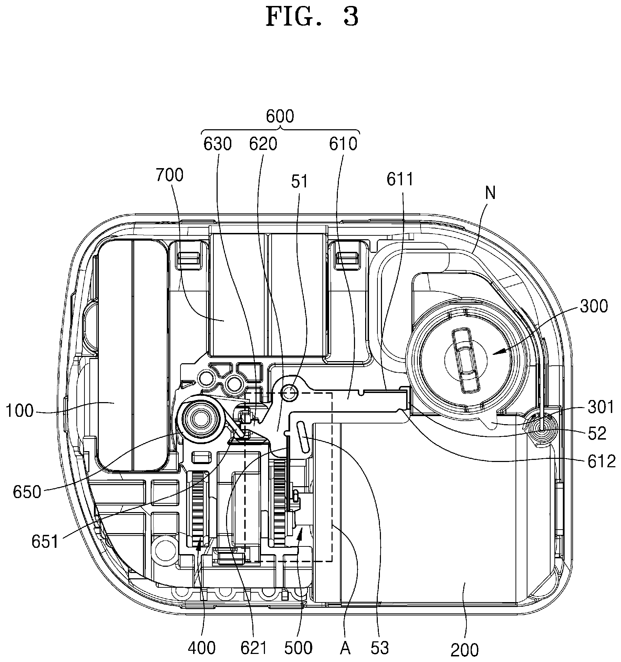

FIG. 2 is a perspective view of an internal arrangement of the drug injection device 1 of FIG. 1 according to an embodiment, and FIG. 3 is a plan view of the drug injection device 1 of FIG. 2.

Referring to FIGS. 1 to 3, an embodiment of the drug injection device 1 may include an outer housing 5 covering the outside of the drug injection device 1, and an attachment portion 6 positioned adjacent to the skin of the user. The drug injection device 1 includes a plurality of parts arranged in an inner space between the outer housing 5 and the attachment portion 6. A bonding portion may be further additionally arranged between the attachment portion 6 and the skin of the user, and the drug injection device 1 may be fixed to the skin by the bonding portion.

The drug injection device 1 may include a base body 50, a pump module 100, the reservoir 200, a needle assembly 300, a driving unit 400, a clutch unit 500, a trigger member 600, and a battery 700.

The base body 50 forms a basic frame of the outer housing 5 and is mounted in the inner space of the outer housing 5. A plurality of base bodies 50 may be included. In an embodiment, the base body 50 may include a first body 50a covering upper portions of internal parts and a second body 50b covering lower portions of the internal parts. The first body 50a and the second body 50b may be assembled to fix the internal parts of the drug injection device 1 at a preset position. In another embodiment, the base body 50 may be formed as an integral single frame.

The base body 50 may provide a space in which the trigger member 600 may rotate. The base body 50 may support the trigger member 600, and the trigger member 600 may be pivoted with respect to a pivot shaft 51 protruding from the base body 50.

The base body 50 may include a stopper that limits a pivot distance of the trigger member 600. A plurality of stoppers may be provided and may limit a movement distance of the trigger member 600 such that the trigger member 600 is pivoted to a preset point. According to an embodiment, the stoppers may include a first stopper 52 and a second stopper 53.

The first stopper 52 protrudes upward from the base body 50 and is arranged adjacent to the needle assembly 300. The first stopper 52 is arranged to contact a first end 610 of the trigger member 600, and may limit a rotational direction and a rotation distance of the first end 610 to prevent the first end 610 from rotating in an opposite direction after rotating in one direction.

In detail, a surface of the first stopper 52, the surface being in contact with the trigger member 600, may be formed to protrude obliquely. When the first end 610 of the trigger member 600 is pivoted in one direction, the first end 610 is moved along an upper surface of the first stopper 52. The upper surface of the first stopper 52 guides movement of the trigger member 600, thus allowing the needle assembly 300 to pivot the trigger member 600 smoothly via rotation.

The first stopper 52 may limit a pivot direction of the trigger member 600. A sidewall of the first stopper 52 may extend from the upper surface thereof and be formed to be substantially perpendicular to a plane of the base body 50. The sidewall of the first stopper 52 prevents the needle assembly 300 and the trigger member 600 from rotating in an opposite direction after the trigger member 600 is rotated by a preset rotation distance in one direction, thereby ensuring the stability of the drug injection device 1.

The second stopper 53 is arranged adjacent to the reservoir 200, the driving unit 400, and the clutch unit 500. The second stopper 53 may be arranged to protrude upward from the base body 50, thereby limiting a movement distance of the second end 620 of the trigger member 600. In an embodiment, the second stopper 53 may have a longitudinal extension line passing through a center of the pivot shaft 51.

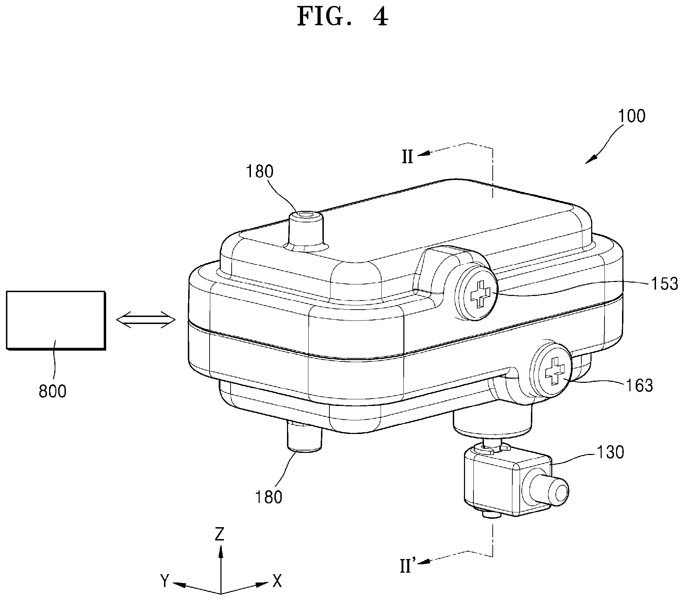

FIGS. 4 to 7B are views for describing an operation of a pump module according to the present disclosure.

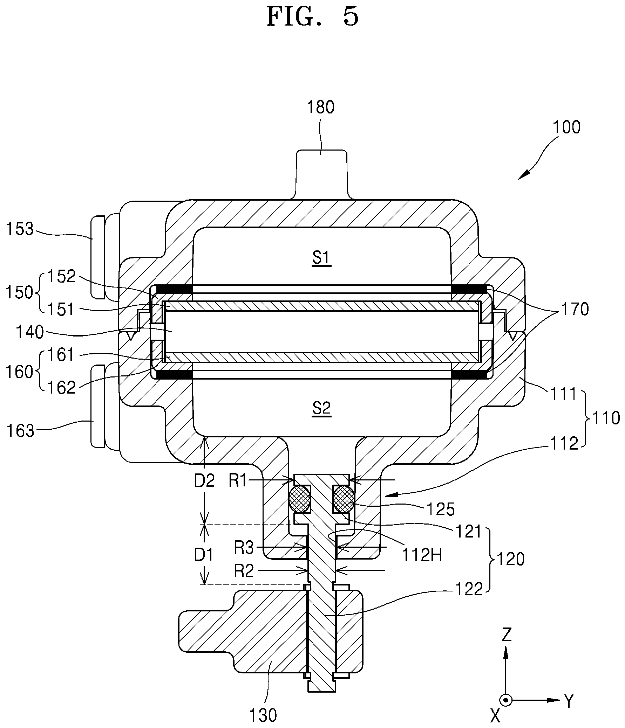

In particular, FIG. 4 is a perspective view of the pump module 100 according to an embodiment of the present disclosure, and FIG. 5 is a cross-sectional view of the pump module 100 taken along line II-II' of FIG. 4.

Referring to FIG. 4, the overall operation of the pump module 100 according to the present disclosure is performed by a controller 800. In detail, the controller 800 determines a driving time, an additional driving time, etc. of the pump module 100 based on information sensed by the pump module 100, and may control a pump driving voltage time of the pump module 100 based on a determination result.

Here, while FIG. 4 illustrates that the controller 800 is implemented as a separate component from the pump module 100, according to another example of the present disclosure, the controller 800 may be implemented as a component included in the pump module 100. When the controller 800 is implemented as a separate component from the pump module 100, the controller 800 may be included in the drug injection device 1 and may be included in the remote device 2 to communicate with the drug injection device 1 through a communication network.

Examples of the communication network may include wired networks such as LAN), WAN), MANs, ISDNs, and wireless networks such as wireless LANs, CDMA, Bluetooth, and satellite communication, or the like, but the present disclosure is not limited thereto.

Also, the controller 800 may be implemented as a digital signal processor (DSP), a microprocessor, or a time controller (TCON) for processing a digital signal. However, the present disclosure is not limited thereto, and the controller 800 may include one or more among a central processing unit (CPU), a micro controller unit (MCU), a micro processing unit (MPU), a controller, an application processor (AP), or a communication processor (CP), an Advanced RISC Machines (ARM) processor, or may be defined by a relevant term. In addition, the controller 800 may be implemented as a system on chip (SoC), large scale integration (LSI), or a field programmable gate array (FPGA) type, in each of which having a processing algorithm embedded therein.

Referring to FIGS. 4 and 5, an inner housing 110 of the pump module 100 may include a shaft hole 112H provided in one side thereof, and a shaft 120 having a certain length may extend to the outside of the inner housing 110 through the shaft hole 112H. In an embodiment, the shaft hole 112H may be formed in a protrusion 112 extending to one side with respect to a main body 111 of the inner housing 110, and a diameter of the protrusion 112 may be less than a diameter of the main body 111.

A first portion 121 of the shaft 120 is arranged inside the inner housing 110, and a second portion 122 thereof extends to the outside of the inner housing 110 through the shaft hole 112H as described above. The shaft 120 may reciprocate in a vertical direction (z-direction) in FIGS. 4 and 5. When the shaft 120 reciprocates, the first portion 121 may linearly reciprocate in an inner space of the inner housing 110, for example, an inner space corresponding to the protrusion 112. A diameter R1 of the first portion 121 of the shaft 120 is greater than a diameter R3 of the shaft hole 112H, and thus, the first portion 121 does not fall out of the inner housing 110.

The second portion 122 of the shaft 120 has a diameter R2 that is less than the diameter R3 of the shaft hole 112H, and to prevent the second portion 122 from falling out of the shaft hole 112H, the second portion 122 may be coupled to a power transmission unit 130 arranged outside the inner housing 110.

A first sealing material 125 may be arranged on a side surface of the first portion 121 of the shaft 120. The inner space of the inner housing 110, for example, a space defined by an inner surface of the inner housing 110 and an inner surface of the shaft 120 is a closed space, and there is a fluid in the inner space, and the first sealing material 125 may prevent fluid from leaking (escape) through a gap between the inner housing 110 and the shaft 120. In FIG. 5, the fluid is omitted for convenience of description.

According to an embodiment, as illustrated in FIG. 5, the first sealing material 125 may cover the side surface of the first portion 121 in a form of an O-ring, and through the first sealing material 125, the fluid inside the inner housing 110 may be prevented from leaking out of (escaping from) the inner housing 110 through the shaft hole 112H. The leakage of the fluid may be further prevented by making a first distance D1 from the first portion 121 of the shaft 120 to the power transmission unit 130 equal to or less than an inner length D2 of the protrusion 112.

A membrane 140 may be arranged in the inner space of the inner housing 110, for example, an inner space corresponding to the main body 111. The inner space includes a first space S1 and a second space S2 respectively located on both sides of the membrane 140 as a center. In FIG. 2, a space that is farther from the shaft 120 with respect to the membrane 140 is referred to as the first space S1, and a space closer to the shaft 120 with respect to the membrane 140 is referred to as the second space S2.

The membrane 140 may have a porous structure in which fluid and ions may move. The membrane 140 may be, for example, a frit-type membrane manufactured by thermally calcining spherical silica. For example, the spherical silica used to form the membrane may have a diameter of about 20 nm to about 500 nm, specifically, a diameter of about 30 nm to about 300 nm, and more specifically, a diameter of about 40 nm to about 200 nm. When the diameter of the spherical silica satisfies the ranges described above, a pressure caused by a first fluid passing through the membrane 140, that is, a sufficient pressure to move the shaft 120, may be generated.

While it has been described that the membrane 140 includes spherical silica in the above embodiment, the membrane 140 is not limited thereto. In another embodiment, as long as the membrane 140 includes a material capable of causing an electrokinetic phenomenon by zeta potential, such as porous silica or porous alumina, the type of the material of the membrane 140 is not limited.

The membrane 140 may have a thickness of about 20 $\mu$m to about 10 mm, specifically, a thickness of about 300 $\mu$m to about 5 mm, and more specifically, a thickness of about 1,000 $\mu$m to about 4 mm.

A first electrode body 150 and a second electrode body 160 are respectively arranged on both sides of the membrane 140. The first electrode body 150 may include a first porous plate 151 and a first electrode strip 152 arranged on a first side of the membrane 140. The second electrode body 160 may include a second porous plate 161 and a second electrode strip 162 arranged on a second side of the membrane 140.

The first and second porous plates 151 and 161 may be arranged to contact both main surfaces of the membrane 140, respectively. The first and second porous plates 151 and 161 may effectively move fluids and ions through the porous structure. The first and second porous plates 151 and 161 may have a structure in which an electrochemical reactant is formed on a porous base layer. The electrochemical reactant may be formed by, for example, electrodeposition or coating on the porous base layer through a method such as electroless plating, vacuum deposition, coating, or a sol-gel process.

The porous base layer may include an insulator. For example, the porous base layer may include at least one selected from a non-conductive ceramic, a non-conductive polymer resin, non-conductive glass, and a combination thereof.

The non-conductive ceramic may include, for example, at least one selected from rock wool, gypsum, ceramics, cement, and combinations thereof, and specifically, at least one selected from rock wool, gypsum, and combinations thereof, but is not limited thereto.

The non-conductive polymer resin may include, for example, at least one selected from: synthetic fibers such as those selected from polypropylene, polyethylene terephthalate, polyacrylonitrile, and combinations thereof; natural fibers such as those selected from wool, cotton, and combinations thereof; sponge; a porous material derived from a living organism, such as a bone of an organism; and combinations thereof, but is not limited thereto.

The non-conductive glass may include at least one selected from glass wool, glass frit, porous glass, and combinations thereof, but is not limited thereto.

The porous base layer may have a pore size of about 0.1 $\mu$m to about 500 $\mu$m, specifically, a pore size of about 5 $\mu$m to about 300 $\mu$m, and more specifically, a pore size of about 10 $\mu$m to about 200 $\mu$m. When the pore size of the porous support satisfies the above-described range, fluid and ions may be effectively moved, thereby improving the stability, lifespan, and efficiency of the pump module 100.

The electrochemical reactant may include a material that may make a pair of reactions in which an oxidation electrode and a reduction electrode exchange positive ions, for example, hydrogen ions, during an electrode reaction of the first and second electrode bodies 150 and 160, and that may constitute, at the same time, a reversible electrochemical reaction. The electrochemical reactant may include at least one selected from, for example, silver/silver oxide, silver/silver chloride, MnO(OH), polyaniline, polypyrrole, polythiophene, polythionine, quinone-based polymer and combinations thereof.

The first and second strips 152 and 162 may be arranged on edges of the first and second porous plates 151 and 161, respectively, and may be connected to the first and second terminals 153 and 163 outside the inner housing 110, respectively. The first and second strips 152 and 162 may include a conductive material such as silver or copper.

The fluid provided in the inner space of the inner housing 110 may include a first fluid and a second fluid having different phases. The first fluid may include a liquid such as water, and the second fluid may include a gas such as air. The first fluid existing in the inner space does not entirely fill the inner space. That is, a volume of the inner space is greater than a volume of the first fluid existing in the inner space. The second fluid is present in a portion of the inner space in which water does not exist.

A second sealing material 170 is arranged on both sides of the structures of the membrane 140, the first electrode body 150, and the second electrode body 160. The second sealing material 170 may have a ring shape having an area corresponding to edges of the above-described structure. The fluid described above, for example, the first fluid, moves from the first space S1 to the second space S2 or in a reverse direction, along a thickness direction of the membrane 140 to pass through the membrane 140, and here, the second sealing material 170 may block a gap between the inner surface of the inner housing 110 and the structure described above, thereby preventing a liquid from moving into the gap.

The fluid may be introduced into the inner space through an inlet 180 as illustrated in FIG. 4. In an embodiment, after the first fluid is entirely filled into the inner space through the inlet 180 on both sides of the pump module 100, a portion of the first fluid is drawn out to the outside through one of the inlets 180, and then the inlets 180 are closed, thereby allowing the first fluid and the second fluid to be in the inner space of the inner housing 110.

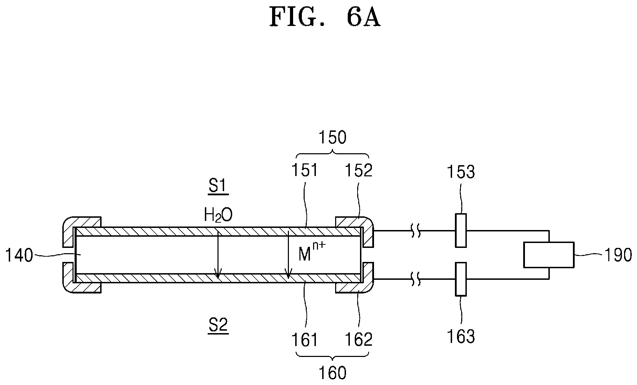

Hereinafter, a behavior of the fluid and movement of the shaft according to the behavior of the fluid are described with reference to FIGS. 6A to 7B.

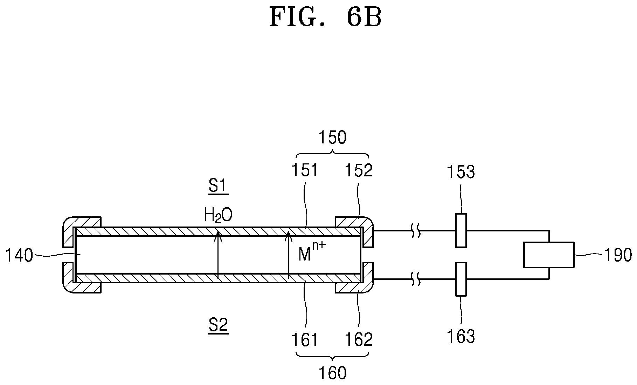

FIGS. 6A and 6B are schematic diagrams illustrating reactions in the first and second electrode bodies with respect to the membrane.

Referring to FIGS. 6A and 6B, the first electrode body 150 and the second electrode body 160 are electrically connected to a power supply unit 190 through the first and second terminals 153 and 163, respectively. By alternately switching and supplying a polarity of a voltage supplied by the power supply unit 190, a direction of movement of a liquid such as water may be changed.

In an embodiment, a case in which silver/silver oxide is used as an electrochemical reactant and the first fluid is a solution containing water is described.

As illustrated in FIG. 6A, when the first electrode body 150 is an oxidation electrode and the second electrode body 160 is a reduction electrode, a reaction of $Ag(s) + H2O \rightarrow 2H+ + 2e-$ takes place, and in the second electrode body 160, a reaction of $Ag2O(s) + 2H+ + 2e- \rightarrow H2O$ takes place.

Positive ions ($Mn+$, for example, hydrogen ions) generated according to the oxidation reaction in the first electrode body 150 pass through the membrane 140 and move toward the second electrode body 160 by a voltage difference. Here, water ($H_2O$) moves together with the positive ions, generating a certain pressure.

Then, as illustrated in FIG. 6B, when the polarity of the voltage supplied by the power supply unit 190 is reversed, an electrochemical reactant previously consumed when the first electrode body 150 is used as the oxidation electrode is recovered when the first electrode body 150 is used as a reduction electrode, and also in a case the first electrode body 150 being used as a reduction electrode, an electrochemical reactant previously consumed when the first electrode body 150 is used as an oxide electrode is recovered. As such, the first and second electrode bodies 150 and 160 may continuously react according to a voltage supply from the power supply unit 200. Unlike in FIG. 3A, when the polarity of the voltage supplied to the first and second electrode bodies 150 and 160 is switched, as illustrated in FIG. 3B, positive ions ($Mn+$, for example, hydrogen ions) and water ($H_2O$) are moved again from the second space S2 to the first space S1.

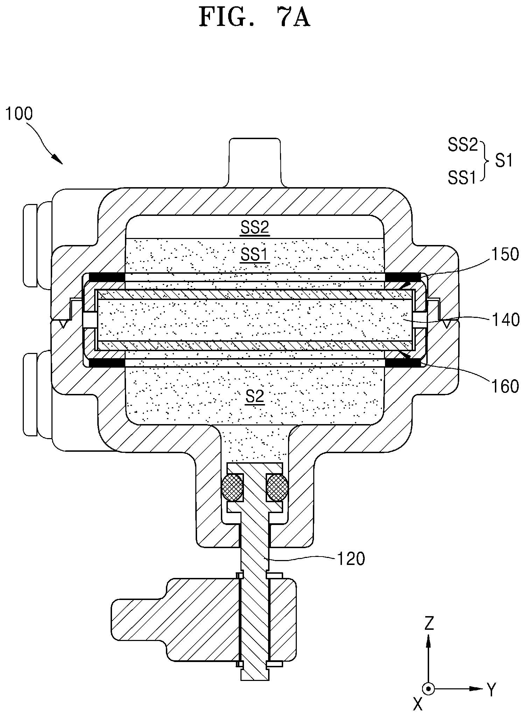

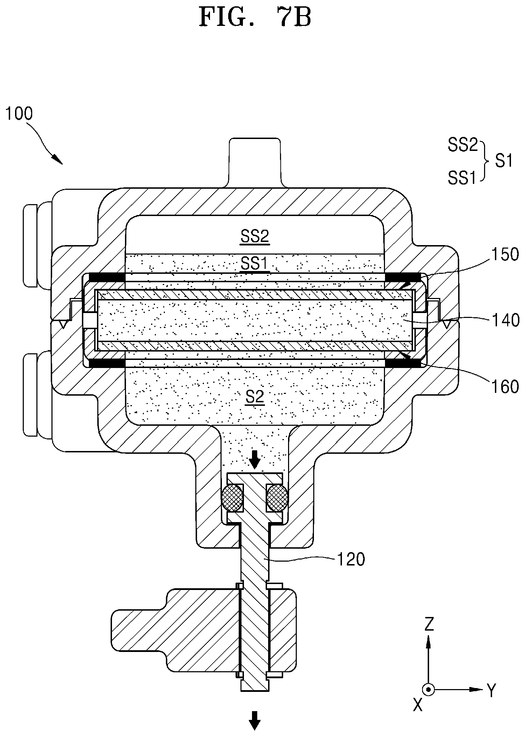

FIGS. 7A and 7B are cross-sectional views illustrating a reciprocating motion of a shaft. FIG. 7A illustrates a state before movement of the shaft, and FIG. 7B illustrates a state after movement of the shaft. FIG. 7A may be understood as a state before a voltage is applied to the first and second electrode bodies 150 and 160 by the power supply unit 190 described above with reference to FIG. 6A.

Referring to FIG. 7A, a first fluid of a liquid such as water exists in the inner space of the inner housing 110, but a volume of the first fluid existing in the inner space is less than the volume of the inner space. A second fluid including a gas such as air exists in a portion of the inner space in which the liquid does not exist.

For example, the first fluid may be in each of the first and second spaces S1 and S2, and the first fluid and the second fluid may coexist in the first space S1, and the volume of the first fluid in the first space S1 may be less than a volume of the first space S1. The first fluid is also present in the second space S2, but unlike the first space S1, the second fluid does not exist in the second space S2. Hereinafter, for convenience of description, a space in the first space S1, in which the first fluid, which is a liquid, exists, is referred to as a first sub-space SS1, and a space of the first space S1, in which the second fluid, which is a gas, exists, is referred to as a second sub-space SS2. The first sub-space SS1 and the second sub-space SS2 may form the first space S1. For example, the remainder of the first space S1 excluding the first sub-space SS1 may be the second sub-space SS2.

In the state of FIG. 7A, when the power supply unit 190 supplies a voltage to the first and second electrode bodies 150 and 160 as described with reference to FIG. 6A, while the reaction described with reference to FIG. 6A takes place, positive ions (for example, hydrogen ions) move along a first direction (-Z direction in FIG. 4) from the first space S1 to the second space S2. Here, as the first fluid (for example, $H_2O$) in the first space S1 together with the positive ions moves along the first direction through the membrane 140, a pressure is generated, and by the pressure, the shaft 120 moves linearly along the first direction, as illustrated in FIG. 4B. While the first fluid (for example, $H_2O$) of the first space S1 moves to the second space S2, a volume ratio of the first sub-space SS1 to the volume of the first space S1 decreases, whereas a ratio of the second sub-space SS2 in the first space S1 increases.

On the contrary, in the state of FIG. 7B, when the power supply unit 190 switches the polarity of the voltage and supplies the voltage to the first and second electrode bodies 150 and 160 as described in FIG. 6B, positive ions (for example, hydrogen ions) and the first fluid (for example, water) move in a second direction (Z-direction in FIG. 4) from the second space S2 to the first space S1, and the shaft 120 is moved again to its original position, as illustrated in FIG. 7A.

When the power supply unit 190 alternately changes the polarity of the voltage applied to the first and second electrode bodies 150 and 160, the shaft 120 may make reciprocating motion of moving in the first direction and then in the second direction opposite to the first direction, and then again in the first direction.

The reciprocating motion of the shaft 120 may be described as a change according to the volume ratio of a space, in which the second fluid exists, in the first space S1, that is, the second sub-space SS2.

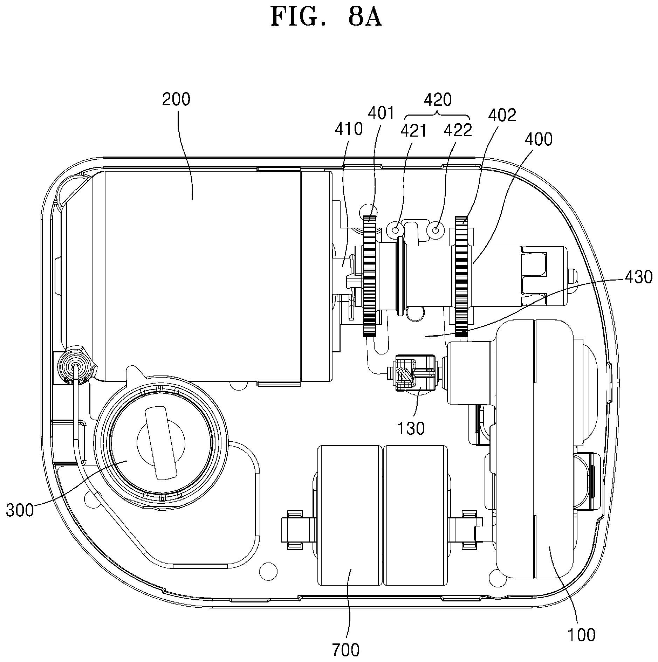

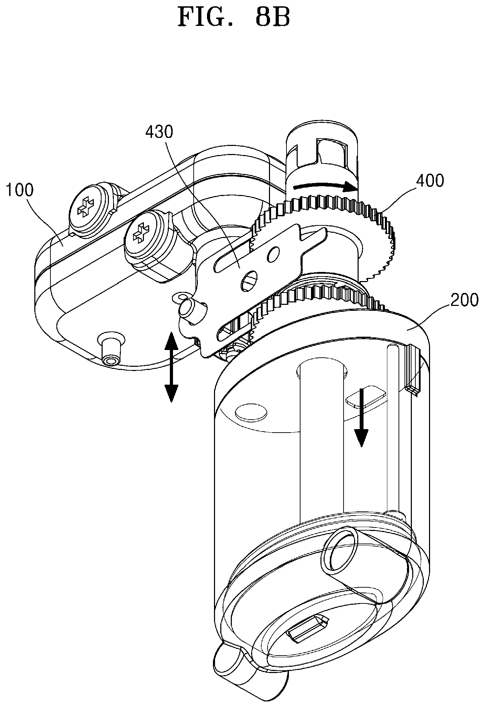

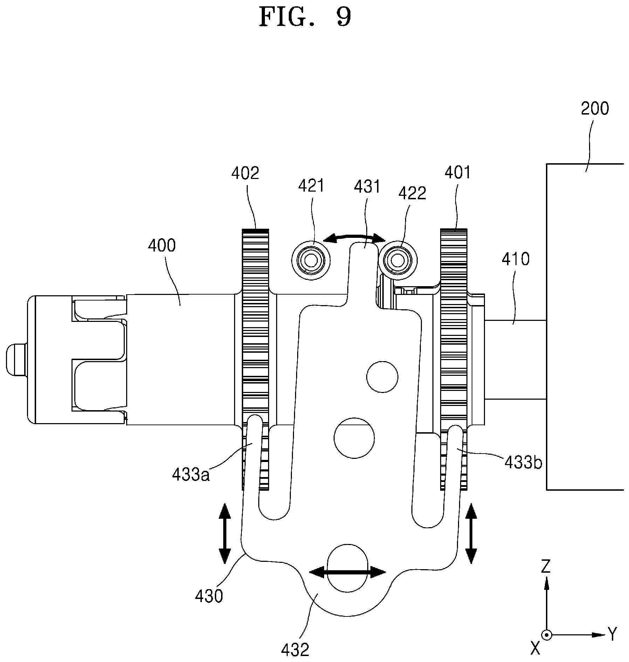

FIG. 8A is a plan view for describing the drug injection device 1 according to an embodiment of the present disclosure, the drug injection device 1 injecting a drug through the pump module 100 and the driving unit 400. FIG. 8B is a perspective view for describing the drug injection device 1 according to an embodiment of the present disclosure, the drug injection device 1 injecting a drug through the pump module 100 and the driving unit 400. FIG. 9 is a plan view for describing an operation of the driving unit 400 according to an embodiment of the present disclosure.

Referring to FIGS. 8A to 9, the power transmission unit 130 may be connected to a rotary part 430. The rotary part 430 may include a second end 431, a first end 432, and third ends 433a and 433b. The second end 431 is a portion that contacts first and second sensors 421 and 422, the first end 432 is a portion connected to the power transmission unit 130, and the third ends 433a and 433b are portions that are in contact with a first connection end 401 and a second connection end 402, respectively.

At least one sensor of first and second sensors 421 and 422 according to an embodiment of the present disclosure may be an anchor sensor, but is not limited thereto, and may be implemented using all types of sensors capable of sensing through a contact operation.

The pump module 100 according to the present disclosure linearly reciprocates the shaft 120 as described with reference to FIGS. 4 to 7B, and accordingly, the power transmission unit 130 connected to the second portion 122 of the shaft 120 may also make linear reciprocating motion in the same direction.

That is, the power transmission unit 130 may be connected to the first end 432 of the rotary part 430, and the first end 432 may also perform linear reciprocating motion according to the linear reciprocating motion of the power transmission unit 130.

As in the example of FIG. 9, in a state in which the second end 431 is in contact with the second sensor 422, when the first end 432 performs linear motion in a right direction (Y-direction) according to the reciprocating motion of the pump module 100, the rotary part 430 performs a rotary motion. While moving in an upward direction (Z-direction), the third end 433b of the rotary part 430 applies a force to a gear of the first connection end 401 to thereby rotate the driving unit 400. In addition, the second end 431 of the rotary part 430 performs a rotary motion to the left (-Y direction) until the second end 431 comes into contact with the first sensor 421, and as the second end 431 comes into contact with the first sensor 421, the rotary motion of the rotary part 430 is stopped.

Similarly, in a state in which the second end 431 is in contact with the first sensor 421, when the first end 432 moves linearly in a left direction (-Y-direction) according to the reciprocating motion of the pump module 100, the rotary part 430 performs a rotary motion. The third end 433a of the rotary part 430 applies a force to a gear of the second connection end 402 while moving in the upward direction (Z-direction) to rotate the driving unit 400. In addition, the second end 431 of the rotary part 430 performs a rotary motion to the right (Y-direction) until the second end 431 comes into contact with the second sensor 422, and as the second end 431 comes into contact with the second sensor 422, the rotary motion of the rotary part 430 is stopped.

As described above, a connection shaft 410 extending from the driving unit 400 in a direction to the reservoir 200 is connected to the driving unit 400 and rotates in response to rotation of the driving unit 400.

That is, as illustrated in FIG. 8B, the linear reciprocating motion in the pump module 100, transmitted through the power transmission unit 130, is converted into a rotary reciprocating motion of the driving unit 400, and the drug injection device 1 according to an embodiment of the present disclosure may be used to inject a drug stored in the reservoir 200 through the rotary reciprocating motion of the driving unit 400.

The controller 800 according to an embodiment of the present disclosure obtains contact time information sensed using the first and second sensors 421 and 422.

Referring to the example of FIG. 9, the controller 800 obtains first contact time information about when the second end 431 which has been in contact with the second sensor 422, is separated from the second sensor 422, and second contact time information about when the second end 431 comes into contact with the first sensor 421. Next, the controller 800 determines a first driving time of the pump module 100 based on the first and second contact time information described above. The first driving time is a driving time for driving a push operation of the pump module 100.

Likewise, the controller 800 obtains first contact time information about when the second end 431 which has been in contact with the first sensor 421, is separated from the first sensor 421, and second contact time information about when the second end 431 comes into contact with the second sensor 422. Next, the controller 800 determines a second driving time of the pump module 100 based on the first and second contact time information described above. The second driving time is a driving time for driving a pull operation of the pump module 100.

The first driving time (push driving time) and the second driving time (pull driving time) described above may be affected by various factors such as membranes, mechanisms, friction force, length, temperature, load, electrolysis, etc., and as the driving time is changed accordingly, an asymmetrical driving time may be caused. When the driving time in a driving direction is asymmetric, gas may be generated inside the pump module 100, and thus, as time passes, the performance thereof is degraded and the product lifespan thereof is shortened.

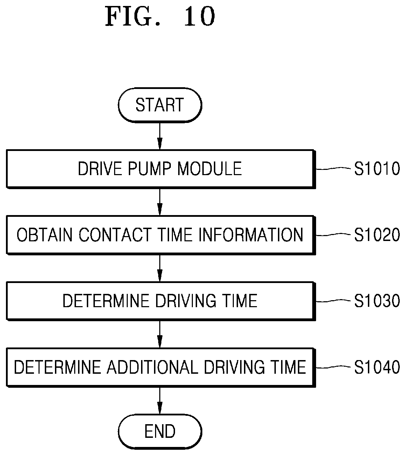

The drug injection device 1 according to the present disclosure may apply an algorithm for symmetrizing driving time of the pump module 100. This will be described in detail with reference to FIGS. 10 to 12.

FIG. 10 is a simple flowchart for describing a driving time symmetrization method of the drug injection device 1,

according to an embodiment of the present disclosure.

The drug injection device 1 may drive a pump by using the pump module 100 in operation S1010. The rotary part 430 performs a reciprocating motion in response to the linear reciprocating motion of the pump module 100, and the drug injection device 1 obtains contact time information through the first and second sensors 421 and 422 and the rotary part 430 in operation S1020. The contact time information is information about a time at which the second end 431 of the rotary part 430 comes into contact with and/or is separated from each of the first sensor 421 and the second sensor 422.

The drug injection device 1 determines a driving time of the pump module 100 based on the contact time information described above in operation S1030. For example, the drug injection device 1 may determine a period of time from a time of the first contact time information, at which the first end 431 is separated from contact with the second sensor 422, to a time of second contact time information, at which the first end 431 comes into contact with the first sensor 421, as a first driving time of the pump module 100. Here, the first driving time is a driving time for driving the push operation of the pump module 100.

Upon determining the first driving time and the second driving time, the drug injection device 1 determines an additional driving time of the pump module 100 based on the above determination in operation S1040. In detail, the drug injection device 1 may determine an additional driving time for each driving time of the push operation and the pull operation. That is, the drug injection device 1 may determine a first additional driving time for the first driving time and a second additional driving time for the second driving time, respectively.

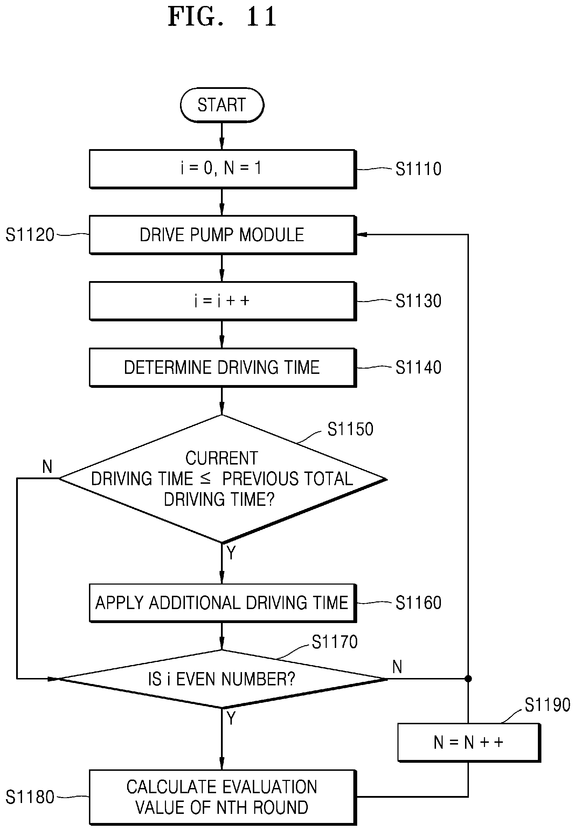

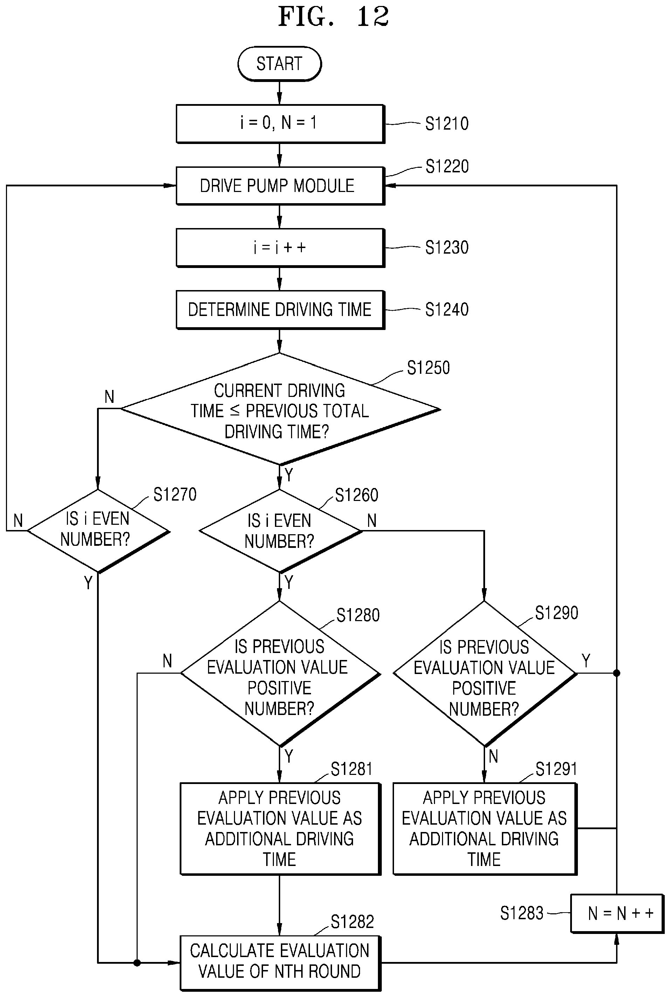

According to an embodiment of the present disclosure, the drug injection device 1 determines an additional driving time after reciprocating motions of the push operation and the pull operation of the pump module 100 are finished. In detail, the drug injection device 1 may determine a current evaluation value indicating a degree of asymmetry in a current driving time of the pump module 100 each time when one reciprocating motion is finished, and may apply an additional driving time of a driving time of a next round, based on the current evaluation value. This will be described in detail with reference to FIGS. 11 and 12.

FIG. 11 is a flowchart of a method, performed by the drug injection device 1 according to an embodiment of the present disclosure, of applying an additional driving time.

Referring to FIG. 11, the drug injection device 1 may set a driving time symmetrization algorithm having initial values of i=0 and N=1 in operation S1110. Here, (i) may refer to a number of times of pump driving, and N may refer to a number of times of pump driving reciprocations.

The drug injection device 1 may drive the pump module 100 in operation S1120 and update the number of times (i) of pump driving to increase the same by one in operation S1130. Next, the drug injection device 1 may determine a driving time of a pump corresponding to a corresponding round in operation S1140. In detail, the drug injection device 1 may determine a driving time of an i-th pump driving based on contact time information obtained using at least one sensor.

The drug injection device 1 may compare a driving time of a current driving with a total driving time of previous drivings, and determine whether the driving time of the current driving is less than or equal to the total driving time of the previous drivings in operation S1150.

For example, the drug injection device 1 may compare a first driving time of a first pump driving of an Nth round with a second total driving time of a second pump driving of an N-1th round. Here, the first pump driving may refer to a push operation of the pump module 100, and in this case, the second pump driving may refer to a pull operation of the pump module 100. However, this is merely an example, and the first pump driving may refer to a pull operation, and the second pump driving may refer to a push operation.

When the first driving time of the first pump driving of the Nth round is less than or equal to the second total driving time of the second pump driving of the N-1th round (S1150-Y), the drug injection device 1 may apply an additional driving time to the first pump driving of the Nth round in operation S1160. On the other hand, when the first driving time of the first pump driving of the Nth round is greater than the second total driving time of the second pump driving of the N-th round (S1150-N), the drug injection device 1 may determine that there is no additional driving time for the first pump driving of the Nth round. This will be described in detail with reference to FIG. 12.

Next, the drug injection device 1 may determine whether the number of times (i) of the pump driving is an even number in operation S1170. When it is determined that the number of times (i) of pump driving is an even number (S1170-Y), the drug injection device 1 may calculate an evaluation value of the N round in operation S1180. After calculating the evaluation value of the N round, the drug injection device 1 may increase the value of N by 1 in operation S1190, and then perform a first pump driving of an N+1th round in operation S1120.

The evaluation value may be a parameter indicating a degree of asymmetry in the driving time of the pump module 100. The evaluation value according to an embodiment of the present disclosure may be calculated through Equations 1 and 2 below.

[Equation 1]

$$\tau_i =$$

$$\begin{cases} \lfloor C(N-1) \rfloor, & \left( C(N-1) < 0 \ \textbf{AND} \ i \in ODD \right) \ \textbf{OR} \ \left( C(N-1) > 0 \ \textbf{AND} \ i \in EVEN \right) \\ 0, & S_i > S_{i-1} \ \textbf{OR} \ i < 2 \ \textbf{OR} \ Otherwise \end{cases}$$

[Equation 2]

$$C(N) = C(N-1) + \left( S_{2N-1} + \tau_{2N-1} \right) - \left( S_{2N} + \tau_{2N} \right)$$

$\tau_i$ may denote an additional driving time of the i-th pump driving. Also, $S_i$ may denote a driving time of the i-th pump driving, the driving time being determined based on contact time information obtained using a sensor. Also, $C(N)$ may be an evaluation value corresponding to the number of times of pump driving reciprocations of the Nth round.

When it is determined that the number of times (i) of pump driving is not an even number (S1170-N), the drug injection device 1 may perform a second pump driving of the Nth round in operation S1120.

FIG. 12 is a flowchart of another method, performed by the drug injection device 1 according to an embodiment of the present disclosure, of applying an additional driving time.

The drug injection device 1 may set a driving time symmetrization algorithm having initial values of i=0 and N=1 in operation S1210. Here, (i) may refer to the number of times of pump driving, and N may refer to the number of times of pump driving reciprocations.

The drug injection device 1 may drive the pump module 100 in operation S1220 and update the number of times (i) of pump driving to increase the same by one in operation S1230. Next, the drug injection device 1 may determine a driving time of a pump of a corresponding round in operation S1240. In detail, the drug injection device 1 may determine a driving time of an i-th pump driving based on contact time information obtained using at least one sensor.

The drug injection device 1 may compare a driving time of a current driving with a total driving time of previous drivings, and determine whether the driving time of the current driving is less than or equal to the total driving time of the previous drivings in operation S1250.

When a first driving time of a first pump driving of an Nth round is greater than a second total driving time of a second pump driving of an N-t1 th round (S1250-N), the drug injection device 1 may determine that there is no additional driving time for the first pump driving of the Nth round. Here, when (i) is an even number (S1270-Y), the drug injection device 1 may calculate an evaluation value of the Nth round in operation S1282. Next, the drug injection device 1 may increase the value of N by 1 in operation S1283, and then perform a first pump driving of an N+1th round in operation S1220. On the other hand, when (i) is not an even number (S1270-N), the drug injection device 1 may perform a second pump driving of the Nth round in operation S1220.

When the first driving time of the first pump driving of the Nth round is less than or equal to the second total driving time of the second pump driving of the N-1th round (S1250-Y), the drug injection device 1 may determine whether (i) is an even number in operation S1260.

When (i) is an even number (S1260-Y), the drug injection device 1 may determine whether an N-1th evaluation value is a positive number in operation S1280. When the N-1th evaluation value is a positive number (S1280-Y), the drug injection device 1 may apply the N-1th evaluation value as the additional driving time for the i-th pump driving. On the other hand, when the N-1th evaluation value is a negative number (S1280-N), the drug injection device 1 may determine that there is no additional driving time for the i-th pump driving. Here, the evaluation value may be a parameter representing a difference between a sum of first pump driving times and a sum of second pump driving times, that is, the degree of asymmetry.

When a criterion of an evaluation value according to an embodiment of the present disclosure is first pump driving, and a case in which the evaluation value is a negative number indicates that the sum of first pump driving times from the first round to the Nth round is less than the sum of second pump driving times from the first round to the Nth round, that is, that an asymmetry has occurred. Similarly, the evaluation value being a positive number indicates that the sum of first pump driving times from the first round to the Nth round is greater than the sum of second pump driving times from the first round to the Nth round, that is, that an asymmetry has occurred.

This is merely an example, and according to another embodiment of the present disclosure, the criterion of the evaluation value may be second pump driving, and in this case, the evaluation value being a positive number indicates that the sum of first pump driving times from the first round to the Nth round is less than the sum of second pump driving times from the first round to the Nth round, that is, that an asymmetry has occurred.

When the i-th pump driving, that is, the second pump driving of the Nth round is finished, the drug injection device 1 may calculate an Nth evaluation value from Equation 1 and Equation 2 in operation S1282. In detail, the drug injection device 1 may calculate the Nth evaluation value based on a total driving time of the first pump driving of the Nth round, a total driving time of the second pump driving of the Nth round, and the evaluation value of the N-1th round the Nth evaluation value. In further detail, the drug injection device 1 may calculate, as the evaluation value of the Nth round, a value obtained by adding the evaluation value of the N-1th round the Nth evaluation value to a value obtained by subtracting the second total driving time of the second pump drivings of the Nth round from the total driving time of the first pump driving of the Nth round. Next, the drug injection device 1 may increase the value of N in operation S1283, and perform the first pump driving of the N+1th round in operation S1220.

When (i) is an odd number (S1260-N), also, the drug injection device 1 may determine whether the N-1th evaluation value is a positive number in operation S1290. When the N-1th evaluation value is a positive number (S1290-Y), the drug injection device 1 may determine that there is no additional driving time for the i-th pump driving, and the drug injection device 1 may drive the pump module 100 to perform second pump driving of the Nth round in operation S1220.

When the N-1th evaluation value is a negative number (S1280-Y), on the other hand, the drug injection device 1 may apply an absolute value of the N-1th evaluation value as an additional driving time for the i-th pump driving in operation S1291. Next, the drug injection device 1 may drive the pump module 100 to perform the second pump driving of the Nth round in operation S1220.

Referring to Equation 1 and Equation 2, the drug injection device 1 according to an embodiment of the present disclosure may determine an evaluation value to be 0 for a first pump driving of a first round (N=1). Here, the drug injection device 1 may determine that there is no additional driving time for a driving time of the first pump driving of the first round and a driving time of a second pump driving of the first round. That is, a first total driving time of the first round may be a first driving time of the first round, and a second total driving time of the first round may be a second driving time of the first round.

For example, application of the driving time symmetrization algorithm according to an embodiment of the present disclosure is as shown below.

Driving time = {(2, 3), (3, 3), (4, 2), (2, 3), (2, 3)}
Total driving time = {(2, 3), (4, 3), (4, 2), (2, 3), (2, 3)}
Evaluation value = {-1, 0, 2, 1, 0}

In detail, the drug injection device 1 may determine an evaluation value to be 0 for the pump driving of the first round (N=1), and as the evaluation value is 0, there is no additional driving time for the driving time of the first pump driving of the first round and the driving time of the second pump driving of the first round. Thus, after performing a reciprocating pump driving of the first round, an evaluation value of the first round is -1 (evaluation value = 2 - 3 + 0).

That is, the evaluation value starts with -1 in a reciprocating pump driving of a second round. A previous total driving time refers to a total driving time of the second pump driving of a reciprocating motion of the first round (N=1). A current total driving time is the first driving time because the additional driving time is not calculated at the time of comparison. Thus, the current total driving time is 3 and the previous total driving time is 3. That is, the current total driving time is the same as the previous total driving time, which is a condition for applying the additional driving time. When the evaluation value is a negative number, it means that the first driving time, which serves as a criterion, is insufficient, and thus, an additional driving time of 1 second is applied. Thus, a first total driving time of the second round is changed to 4 seconds. With regard to a second pump driving of the second round, the current total driving time is 3 and the previous total driving time is 4, that is, the current total driving time is less than the previous total driving time. This may be a condition for applying an additional driving time, but the evaluation value is -1, which means that the driving time exceeds 1 second in the second pump driving, and thus, an additional driving time is not applied. Thus, the total driving time remains unchanged and as 3 seconds. When the reciprocating pump driving of the second round is finished, the evaluation value may be recalculated. Here, an evaluation value of the second round is 0 (evaluation value = 4 - 3 + (-1)).

In a reciprocating pump driving of a third round, the evaluation value starts with zero. That is, as the evaluation value is 0, there is no additional driving time in the case of the pump driving of the third round. After the reciprocating pump driving of the third round, the evaluation value is 2 (evaluation value = 4 - 2 + 0).

In a reciprocating pump driving of a fourth round, the evaluation value starts with 2. A first driving time of a first pump driving of the fourth round is 2, and a previous total driving time is 2. A current total driving time is equal to the previous total driving time, which is a condition for applying an additional driving time, but as the evaluation value is a positive number, there is no additional driving time for the first driving time. On the other hand, in the case of a second pump driving of the fourth round, a current second driving time is 3 and a previous total driving time thereof is 2, and thus, the current total driving time is greater than the previous total driving time, and there is no additional driving time, accordingly. After the reciprocating pump driving of the fourth round, the evaluation value is 1 (evaluation value = 2 - 3 + 2).

In a reciprocating pump driving of a fifth round, the evaluation value starts with 1. A first driving time of a first pump driving of the fifth round is 2, and a previous total driving time thereof is 3. A current total driving time is less than the previous total driving time, but as the evaluation value is a negative number, there is no additional driving time for the first driving time. Thereafter, a second driving time of a second pump driving of the fifth round is 3, and the previous total driving time is 2, that is, a current total driving time is greater than the previous total driving time, and thus there is no additional driving time. After the reciprocating pump driving of the fifth round is finished, the evaluation value is 0 (evaluation value = 2 - 3 + 1).

After the reciprocating pump driving of the fifth round, a sum of the first driving times and a sum of the second driving times of each round are both 14 seconds. As in the above example, the evaluation value is used as a parameter representing the degree of asymmetry, and by applying an additional driving time through the above-described algorithm, the driving time is balanced.

According to the driving time symmetrization algorithm of the present disclosure, gas inside an electroosmotic pressure-based actuator is not generated due to the symmetrical driving time, thus increasing the lifespan of the actuator.

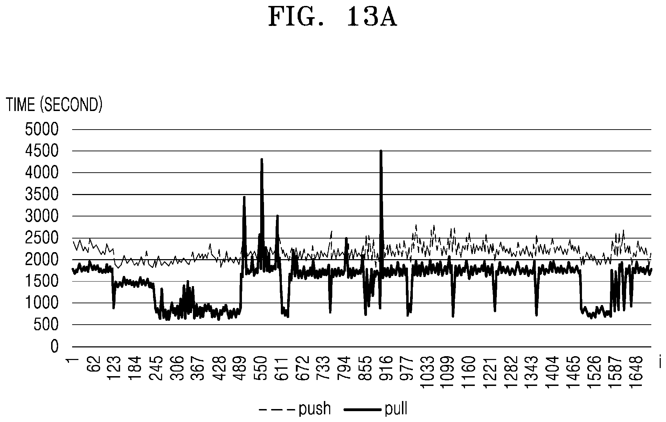

FIGS. 13A to 13C illustrate the effect of symmetrical driving time of a pump module, to which a symmetrization algorithm according to an embodiment of the present disclosure is applied.

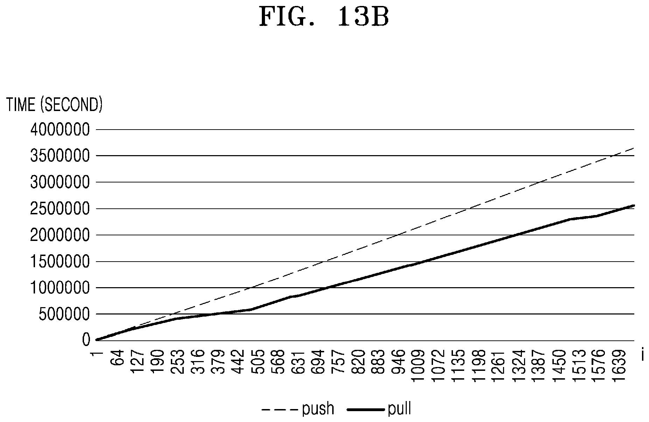

FIG. 13A is a graph showing driving times for each number of times of driving of a pull operation and a push operation before applying the driving time symmetrization algorithm according to the present disclosure. Referring to FIG. 13B, when accumulated driving times of the pull operation and the push operation before the driving time symmetrization algorithm of the present disclosure is applied are compared with each other, the pump module is driven gradually asymmetrically as the number of times of pump driving increases.

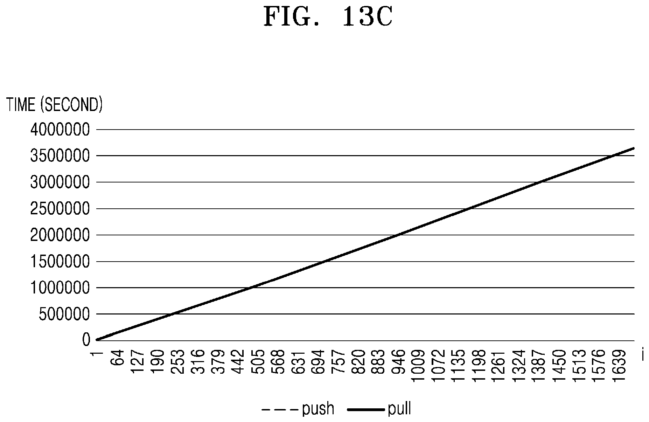

On the other hand, referring to FIG. 13C, when the driving time symmetrization algorithm according to the present disclosure is applied, it can be seen that the accumulated driving times of the pull operation and the push operation increase symmetrically even when the number of times of driving increases. As described above, according to the driving time symmetrization algorithm of the present disclosure, the pump module is driven with a symmetrical driving time.

The methods according to various embodiments of the present disclosure described above may be implemented in the form of an application that can be installed in an existing electronic device.

In addition, the methods according to various embodiments of the present disclosure described above may be implemented only by software upgrade or hardware upgrade of an existing electronic device.

In addition, various embodiments of the present disclosure described above may be performed through an embedded server provided an electronic device or an external server of the electronic device.

According to an embodiment of the present disclosure, the various embodiments described above may be implemented, by using software, hardware, or a combination thereof, as software including instructions stored in a computer-readable recording medium, which is readable by a computer or a similar device thereto. In some cases, the embodiments described herein may be implemented by a processor itself. According to the software implementation, embodiments such as procedures and functions described herein may be implemented as separate software modules. Each of the software modules may perform one or more functions and operations described herein.

Meanwhile, a computer or a similar device thereto may include a device capable of calling a stored command from a storage medium and operating according to the called command, and may include the device according to the disclosed embodiments. When the instruction is executed by a processor, the processor may directly perform a function corresponding to the instruction or may perform the function by using other components under the control by the processor. The instructions may include code generated or executed by a compiler or interpreter.

A machine-readable recording medium is provided in a form of a non-transitory computer-readable recording medium. The term 'non-transitory' only means that a storage medium does not include a signal and is tangible, and does not distinguish whether data is semi-permanently or temporarily stored in the storage medium. In this case, the non-transitory computer-readable recording medium refers to a medium that stores data semi-permanently, rather than a medium that stores data temporarily, such as a register, cache, memory, etc., and can be read by a device. Examples of the non-transitory computer-readable recording medium may include a CD, DVD, hard disk, Blu-ray disk, USB, memory card, ROM, and the like.

While the present disclosure has been particularly shown and described with reference to embodiments thereof illustrated in the drawings, it will be understood by those skilled in the art that various changes in form and details may be made. Therefore, the scope of the present disclosure shall be defined by the appended claims.

**Claims**

1. A drug injection device comprising:

   an electro-osmotic pressure-based actuator (100) including a shaft (120) configured to perform a linear reciprocating motion in one direction defining a push operation and a pull operation of the actuator (100);
   a first sensor (421) and a second sensor (422);
   a driving unit (400) including a first connection end (401) and a second connection end (402); and
   a rotary part (430) including a first end (432) connected to the shaft (120) so that the rotary part (430) is configured to rotate and reciprocate according to the linear reciprocating motion, a second end (431) reciprocally movable between a state in which the second end (431) is in contact with the first sensor (421) and the push operation stops and a state in which the second end (431) is in contact with the second sensor (422) and the pull operation stops, and two third ends (433a, 433b) which are portions that are in contact with the first connection end (401) and the second connection end (402), respectively, wherein:

each third end (433a, 433b) is configured to apply a force, while moving in an upward direction of the reciprocating motion, to a respective gear of the first connection end (401) and the second connection end (402), respectively, to thereby rotate the driving unit (400);

the first sensor (421) is configured to obtain first contact time information about a time when the first sensor (421) comes into contact with the second end (431), and the second sensor (422) is configured to obtain second contact time information about a time when the second sensor (422) comes into contact with the second end (431); and

a controller (800) is configured to determine, based on the first contact time information and the second contact time information, a first driving time for driving the push operation and a second driving time for driving the pull operation of the actuator (100), determine whether or not to apply an additional driving time of the actuator (100) after the push operation stops or after the pull operation stops, based on the first driving time and the second driving time, and control the actuator (100) to perform the linear reciprocating motion in response to the additional driving time.

2. The drug injection device of claim 1, wherein the shaft (120) reciprocates in a first direction from a first space (S1) toward a second space (S2) and in a second direction opposite to the first direction, and
the first space (S1) and the second space (S2) are divided from each other with respect to a membrane (140) located inside the actuator (100), wherein a space farther from the shaft (120) with respect to the membrane (140) is the first space (S1), and a space closer to the shaft (120) is the second space (S2).

3. A non-transitory computer-readable recording medium having recorded thereon a program for executing a method of driving a drug injection device, the drug injection device comprising:

an electro-osmotic pressure-based actuator (100) including a shaft (120) configured to perform a linear reciprocating motion in one direction defining a push operation and a pull operation of the actuator (100);
a first sensor (421) and a second sensor (422);
a driving unit (400) including a first connection end (401) and a second connection end (402); and
a rotary part (430) including a first end (432) connected to the shaft (120) so that the rotary part (430) is configured to rotate and reciprocate according to the linear reciprocating motion, a second end (431) reciprocally movable between a state in which the second end (431) is in contact with the first sensor (421) and the push operation stops and a state in which the second end (431) is in contact with the second sensor (422) and the pull operation stops, and two third ends (433a, 433b) which are portions that are in contact with the first connection end (401) and the second connection end (402), respectively, wherein each third end (433a, 433b) is configured to apply a force, while moving in an upward direction of the reciprocating motion, to a respective gear of the first connection end (401) and the second connection end (402), respectively, to thereby rotate the driving unit (400),
the method including the steps:

obtaining first contact time information about a time when the first sensor (421) comes into contact with the second end (431),
obtaining second contact time information about a time when the second sensor (422) comes into contact with the second end (431); and
determining based on the first contact time information and the second contact time information, a first driving time for driving the push operation and a second driving time for driving the pull operation of the actuator (100),
determining whether or not to apply an additional driving time of the actuator (100) after the push operation stops or after the pull operation stops, based on the first driving time and the second driving time, and
controlling the actuator (100) to perform the linear reciprocating motion in response to the additional driving time.

**Patentansprüche**

1. Arzneimittelinjektionsvorrichtung, umfassend:

einen elektroosmotischen druckbasierten Aktuator (100), umfassend eine Welle (120), die ausgelegt ist, um eine lineare Hin- und Her-Bewegung in eine Richtung durchzuführen, die einen Drückvorgang und einen Ziehvorgang des Aktuators (100) definiert;
einen ersten Sensor (421) und einen zweiten Sensor (422);
eine Antriebseinheit (400), einschließlich eines ersten Verbindungsendes (401) und eines zweiten Verbindungsendes (402); und
einen drehenden Teil (430), einschließlich eines ersten Endes (432), das mit der Welle (120) verbunden ist, sodass der drehende Teil (430) ausgelegt ist, um gemäß der linearen Hin- und Her-Bewegung zu rotieren, und eines zweiten Endes (431), das zwischen einem Zustand, in dem das zweite Ende (431) mit dem ersten Sensor (421) in Kontakt ist und der Drückvorgang anhält, und einem Zustand, in dem das zweite Ende (431) mit dem zweiten Sensor (422) in Kontakt ist und der Ziehvorgang anhält, bewegbar ist, und zwei dritten Enden (433a, 433b), die Abschnitte sind, die mit dem ersten Verbindungsende (401) bzw. dem zweiten Verbindungsende (402) in Kontakt stehen, wobei:

jedes dritte Ende (433a, 433b) ausgelegt ist, um, während es sich in eine Aufwärtsrichtung der Hin- und Her-Bewegung bewegt, eine Kraft auf ein entsprechendes Zahnrad des ersten Verbindungsendes (401) bzw. des zweiten Verbindungsendes (402) anzuwenden, um dadurch die Antriebseinheit (400) zu rotieren;
der erste Sensor (421) ausgelegt ist, um erste Kontaktzeitinformationen über eine Zeit, zu der der erste Sensor (421) mit dem zweiten Ende (431) in Kontakt kommt, zu erhalten, und der zweite Sensor (422) ausgelegt ist, um zweite Kontaktzeitinformationen über eine Zeit, zu der der zweite Sensor (422) mit dem zweiten Ende (431) in Kontakt kommt, zu erhalten; und
eine Steuerung (800) ausgelegt ist, um basierend auf den ersten Kontaktzeitinformationen und den zweiten Kontaktzeitinformationen eine erste Antriebszeit zum Antreiben des Drückvorgangs und eine zweite Antriebszeit zum Antreiben des Ziehvorgangs des Aktuators (100) zu bestimmen, zu bestimmen, ob eine zusätzliche Antriebszeit des Aktuators (100), nachdem der Drückvorgang anhält oder nachdem der Ziehvorgang anhält, basierend auf der ersten Antriebszeit und der zweiten Antriebszeit anzuwenden ist oder nicht, und um den Aktuator (100) zu steuern, um die lineare Hin- und Her-Bewegung als Reaktion auf die zusätzliche Antriebszeit durchzuführen.

2. Arzneimittelinjektionsvorrichtung nach Anspruch 1, wobei sich die Welle (120) in eine erste Richtung von einem ersten Raum (S1) in Richtung eines zweiten Raums (S2) und in eine zweite Richtung entgegengesetzt zur ersten Richtung hin- und herbewegt, und
wobei der erste Raum (S1) und der zweite Raum (S2) in Bezug auf eine Membran (140), die sich innerhalb des Aktuators (100) befindet, voneinander getrennt sind, wobei ein Raum, der in Bezug auf die Membran (140) weiter weg von der Welle (120) ist, der erste Raum (S1) ist, und ein Raum, der näher zur Welle (120) ist, der zweite Raum (S2) ist.

3. Nichtflüchtiges computerlesbares Aufzeichnungsmedium, das darauf aufgezeichnet ein Programm zum Ausführen eines Verfahrens zum Antreiben einer Arzneimittelinjektionsvorrichtung aufweist, wobei die Arzneimittelinjektionsvorrichtung Folgendes umfasst:

ein elektroosmotischer druckbasierter Aktuator (100), umfassend eine Welle (120), die ausgelegt ist, um eine lineare Hin- und Her-Bewegung in eine Richtung durchzuführen, die einen Drückvorgang und einen Ziehvorgang des Aktuators (100) definiert;
einen ersten Sensor (421) und einen zweiten Sensor (422);
eine Antriebseinheit (400), einschließlich eines ersten Verbindungsendes (401) und eines zweiten Verbindungsendes (402); und
einen drehenden Teil (430), einschließlich eines ersten Endes (432), das mit der Welle (120) verbunden ist, sodass der drehende Teil (430) ausgelegt ist, um gemäß der linearen Hin- und Her-Bewegung zu rotieren, und eines zweiten Endes (431), das zwischen einem Zustand, in dem das zweite Ende (431) mit dem ersten Sensor (421) in Kontakt ist und der Drückvorgang anhält, und einem Zustand, in dem das zweite Ende (431) mit dem zweiten Sensor (422) in Kontakt ist und der Ziehvorgang anhält, bewegbar ist, und zwei dritten Enden (433a, 433b), die Abschnitte sind, die mit dem ersten Verbindungsende (401) bzw. dem zweiten Verbindungsende (402) in Kontakt stehen, wobei jedes dritte Ende (433a, 433b) ausgelegt ist, um, während es sich in eine Aufwärtsrichtung der Hin- und Her-Bewegung bewegt, eine Kraft auf ein entsprechendes Zahnrad des ersten Verbindungsendes (401) bzw. des zweiten Verbindungsendes (402) anzuwenden, um dadurch die Antriebseinheit (400) zu rotieren;

wobei das Verfahren die folgenden Schritte umfasst:

Erhalten erster Kontaktzeitinformationen über eine Zeit, zu der der erste Sensor (421) mit dem zweiten Ende (431) in Kontakt kommt,

Erhalten von zweiten Kontaktzeitinformationen über eine Zeit, zu der der zweite Sensor (422) mit dem zweiten Ende (431) in Kontakt kommt; und

Bestimmen einer ersten Antriebszeit zum Antreiben des Drückvorgangs und einer zweiten Antriebszeit zum Antreiben des Ziehvorgangs des Betriebselements (100) basierend auf den ersten Kontaktzeitinformationen und der zweiten Kontaktzeitinformationen,

Bestimmen, ob eine zusätzliche Antriebszeit des Aktuators (100), nachdem der Drückvorgang anhält oder nachdem der Ziehvorgang anhält, basierend auf der ersten Antriebszeit und der zweiten Antriebszeit anzuwenden ist oder nicht, und

Steuern des Aktuators (100), um die lineare Hin- und Her-Bewegung als Reaktion auf die zusätzliche Antriebszeit durchzuführen.

## Revendications

1. Dispositif d'injection de médicament, comprenant :

un actionneur à base de pression électro-osmotique (100) incluant un arbre (120) configuré pour effectuer un mouvement de va-et-vient linéaire dans une certaine direction définissant une opération de poussée et une opération de traction de l'actionneur (100) ;

un premier capteur (421) et un second capteur (422) ;

une unité d'entraînement (400) incluant une première extrémité de connexion (401) et une seconde extrémité de connexion (402) ; et

une partie rotative (430) incluant une première extrémité (432) connectée à l'arbre (120) de telle sorte que la partie rotative (430) soit configurée pour tourner et effectuer un mouvement de va-et-vient selon le mouvement de va-et-vient linéaire, une deuxième extrémité (431) mobile en va-et-vient entre un état dans lequel la deuxième extrémité (431) est en contact avec le premier capteur (421) et l'opération de poussée s'arrête, et un état dans lequel la deuxième extrémité (431) est en contact avec le second capteur (422) et l'opération de traction s'arrête, et deux troisièmes extrémités (433a, 433b) qui sont des parties qui sont en contact avec la première extrémité de connexion (401) et la seconde extrémité de connexion (402), respectivement, dans lequel :

chaque troisième extrémité (433a, 433b) est configurée pour appliquer une force, tout en se déplaçant dans une direction ascendante du mouvement de va-et-vient, à un engrenage respectif de la première extrémité de connexion (401) et de la seconde extrémité de connexion (402), respectivement, pour ainsi faire tourner l'unité d'entraînement (400) ;

le premier capteur (421) est configuré pour obtenir des premières informations de temps de contact concernant un temps où le premier capteur (421) vient en contact avec la deuxième extrémité (431), et le second capteur (422) est configuré pour obtenir des secondes informations de temps de contact concernant un temps où le second capteur (422) vient en contact avec la première extrémité (431) ; et

un dispositif de commande (800) est configuré pour déterminer, sur la base des premières informations de temps de contact et des secondes informations de temps de contact, un premier temps d'entraînement pour entraîner l'opération de poussée et un second temps d'entraînement pour entraîner l'opération de traction de l'actionneur (100), déterminer s'il faut appliquer ou non un temps d'entraînement supplémentaire de l'actionneur (100) après l'arrêt de l'opération de poussée ou après l'arrêt de l'opération de traction, sur la base du premier temps d'entraînement et du second temps d'entraînement, et commander l'actionneur (100) pour effectuer le mouvement de va-et-vient linéaire en réponse au temps d'entraînement supplémentaire.

2. Dispositif d'injection de médicament selon la revendication 1, dans lequel l'arbre (120) effectue un mouvement de va-et-vient dans une première direction à partir d'un premier espace (S1) vers un second espace (S2) et dans une seconde direction opposée à la première direction, et

le premier espace (S1) et le second espace (S2) sont divisés l'un par rapport à l'autre par rapport à une membrane (140) située à l'intérieur de l'actionneur (100), dans lequel un espace plus éloigné de l'arbre (120) par rapport à la membrane (140) est le premier espace (S1), et un espace plus proche de l'arbre (120) est le second espace (S2).

**3.** Support d'enregistrement lisible par ordinateur non transitoire sur lequel est enregistré un programme pour exécuter un procédé de commande d'un dispositif d'injection de médicament, le dispositif d'injection de médicament comprenant :

un actionneur à base de pression électro-osmotique (100) incluant un arbre (120) configuré pour effectuer un mouvement de va-et-vient linéaire dans une certaine direction définissant une opération de poussée et une opération de traction de l'actionneur (100) ;
un premier capteur (421) et un second capteur (422) ;
une unité d'entraînement (400) incluant une première extrémité de connexion (401) et une seconde extrémité de connexion (402) ; et
une partie rotative (430) incluant une première extrémité (432) connectée à l'arbre (120) de telle sorte que la partie rotative (430) soit configurée pour tourner et effectuer un mouvement de va-et-vient selon le mouvement de va-et-vient linéaire, une deuxième extrémité (431) mobile en va-et-vient entre un état dans lequel la deuxième extrémité (431) est en contact avec le premier capteur (421) et l'opération de poussée s'arrête, et un état dans lequel la deuxième extrémité (431) est en contact avec le second capteur (422) et l'opération de traction s'arrête, et deux troisièmes extrémités (433a, 433b) qui sont des parties qui sont en contact avec la première extrémité de connexion (401) et la seconde extrémité de connexion (402), respectivement, dans lequel chaque troisième extrémité (433a, 433b) est configurée pour appliquer une force, tout en se déplaçant dans une direction ascendante du mouvement de va-et-vient, à un engrenage respectif de la première extrémité de connexion (401) et de la seconde extrémité de connexion (402), respectivement, pour ainsi faire tourner l'unité d'entraînement (400),
le procédé incluant les étapes consistant à :

obtenir des premières informations de temps de contact concernant un temps où le premier capteur (421) vient en contact avec la deuxième extrémité (431), obtenir des secondes informations de temps de contact concernant un temps où le second capteur (422) vient en contact avec la première extrémité (431) ; et
déterminer, sur la base des premières informations de temps de contact et des secondes informations de temps de contact, un premier temps d'entraînement pour entraîner l'opération de poussée et un second temps d'entraînement pour entraîner l'opération de traction de l'actionneur (100),
déterminer s'il faut appliquer ou non un temps d'entraînement supplémentaire de l'actionneur (100) après l'arrêt de l'opération de poussée ou après l'arrêt de l'opération de traction, sur la base du premier temps d'entraînement et du second temps d'entraînement, et
commander l'actionneur (100) pour effectuer le mouvement de va-et-vient linéaire en réponse au temps d'entraînement supplémentaire.

# FIG. 1

# FIG. 2

# FIG. 3

# FIG. 4

# FIG. 5

# FIG. 6A

# FIG. 6B

# FIG. 7A

# FIG. 7B

EP 4 079 346 B1

FIG. 8A

26

# FIG. 8B

# FIG. 9

# FIG. 10

```
        ( START )
            |
            v
+---------------------------+
|    DRIVE PUMP MODULE      |——— S1010
+---------------------------+
            |
            v
+---------------------------+
| OBTAIN CONTACT TIME       |——— S1020
| INFORMATION               |
+---------------------------+
            |
            v
+---------------------------+
|  DETERMINE DRIVING TIME   |——— S1030
+---------------------------+
            |
            v
+---------------------------+
| DETERMINE ADDITIONAL      |——— S1040
| DRIVING TIME              |
+---------------------------+
            |
            v
        ( END )
```

# FIG. 11

```
            ( START )
                │
                ▼
    ┌───────────────────────┐
    │     i = 0, N = 1       │──── S1110
    └───────────────────────┘
                │
                ▼
S1120 ──┌───────────────────────┐◄─────────┐
        │    DRIVE PUMP MODULE   │          │
        └───────────────────────┘          │
                │                           │
                ▼                           │
    ┌───────────────────────┐               │
    │       i = i + +        │──── S1130     │
    └───────────────────────┘               │
                │                           │
                ▼                           │
    ┌───────────────────────┐               │
    │ DETERMINE DRIVING TIME │──── S1140     │
    └───────────────────────┘               │
                │                           │
                ▼                           │
              ╱─────╲  S1150                │
      N   ╱   CURRENT    ╲                  │
    ◄────  DRIVING TIME ≤ PREVIOUS TOTAL    │
         ╲    DRIVING TIME?   ╱             │
    │         ╲─────╱                        │
    │            │ Y                         │
    │            ▼                           │
    │  ┌───────────────────────┐             │
    │  │ APPLY ADDITIONAL       │── S1160     │
    │  │ DRIVING TIME           │             │
    │  └───────────────────────┘             │
    │            │                           │
    │            ▼       S1170               │
    │          ╱─────╲        N              │
    └────────  IS i EVEN NUMBER? ────────────┤
               ╲─────╱                       │
                  │ Y              S1190      │
                  │         ┌──────────────┐ │
                  │         │   N = N + +   │ │
                  │         └──────────────┘ │
                  ▼                           │
    ┌───────────────────────┐                 │
S1180│ CALCULATE EVALUATION  │────────────────┘
    │ VALUE OF NTH ROUND     │
    └───────────────────────┘
```

30

# FIG. 12

START

i = 0, N = 1 — S1210

DRIVE PUMP MODULE — S1220

i = i + + — S1230

DETERMINE DRIVING TIME — S1240

CURRENT DRIVING TIME ≤ PREVIOUS TOTAL DRIVING TIME? — S1250

IS i EVEN NUMBER? — S1270

IS i EVEN NUMBER? — S1260

IS PREVIOUS EVALUATION VALUE POSITIVE NUMBER? — S1280

IS PREVIOUS EVALUATION VALUE POSITIVE NUMBER? — S1290

APPLY PREVIOUS EVALUATION VALUE AS ADDITIONAL DRIVING TIME — S1281

APPLY PREVIOUS EVALUATION VALUE AS ADDITIONAL DRIVING TIME — S1291

S1283 — N = N + +

CALCULATE EVALUATION VALUE OF NTH ROUND — S1282

# FIG. 13A

# FIG. 13B

# FIG. 13C

TIME (SECOND)

---push ——pull

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007104596 A1

- WO 2008107467 A1